(19) Europäisches Patentamt
European Patent Office
Office européen des brevets

(11) Veröffentlichungsnummer: **0 000 508**
**B1**

(12) # EUROPÄISCHE PATENTSCHRIFT

(45) Veröffentlichungstag der Patentschrift: **02.09.81**

(21) Anmeldenummer: **78100376.9**

(22) Anmeldetag: **12.07.78**

(51) Int. Cl.³: **C 07 D 213/64,**
**A 01 N 43/40**

(54) **Phenylacetate von 2-Oxy-Pyridyl, Verfahren zu deren Herstellung und deren Verwendung als Schädlingsbekämpfungsmittel.**

(30) Priorität: **20.07.77 CH 8999/77**
**07.12.77 CH 15003/77**
**26.05.78 CH 5777/78**

(43) Veröffentlichungstag der Anmeldung:
**07.02.79 Patentblatt 79/3**

(45) Bekanntmachung des Hinweises auf die Patenterteilung:
**02.09.81 Patentblatt 81/35**

(84) Benannte Vertragsstaaten:
**BE CH DE FR GB NL**

(56) Entgegenhaltungen:
**FR - A - 2 241 533**

(73) Patentinhaber: **CIBA-GEIGY AG**
**Patentabteilung Postfach**
**CH-4002 Basel (CH)**

(72) Erfinder: **Kristiansen, Odd, Dr.**
**Ringweg 12**
**CH-4313 Möhlin (CH)**
Erfinder: **Ackermann, Peter, Dr.**
**Reichensteinerstrasse 12**
**CH-4153 Reinach (CH)**
Erfinder: **Drabek, Jozef, Dr.**
**Benkenstrasse 12**
**CH-4104 Oberwil (CH)**
Erfinder: **Farooq, Saleem, Dr.**
**Im Schaiengarten**
**CH-4107 Ettingen (CH)**
Erfinder: **Gsell, Laurenz, Dr.**
**Poolstrasse 26a**
**CH-4414 Füllinsdorf (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 508 B1

# Phenylacetate von 2-Oxy-Pyridyl, Verfahren zu deren Herstellung und deren Verwendung als Schädlingsbekämpfungsmittel

Die vorliegende Erfindung betrifft Phenylacetate von 2-Oxy-Pyridyl und ihre Salze mit anorganischen und organischen Säuren, Verfahren zu ihrer Herstellung und ihre Verwendung in der Schädlingsbekämpfung.

In der französischen Patentschrift Nr. 2 241 533 sind insektizide Phenylacetate beschreiben.

Die erfindungsgemässen Phenylacetate der Formel I unterscheiden sich von den bekannten Verbindungen durch den Ersatz des Phenylteiles des Phenoxyrestes durch einen Pyridylrest und sind demgegenüber, wegen dem Pyridyloxyrest, zur Salzbildung befähigt.

Die Phenylacetate haben die Formel

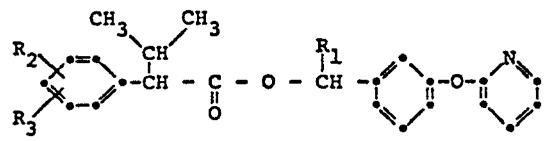

(I)

worin $R_1$ Wasserstoff, Cyano, Aethinyl oder Methyl und $R_2$ und $R_3$ je Wasserstoff, Halogen, $C_1$—$C_4$-Alkyl oder $C_1$—$C_4$—Alkoxy bedeuten.

Für die Salzbildung kommen anorganische Säuren wie beispielsweise HCl, $H_2SO_4$, HBr und $H_3PO_4$ und als organische Säuren beispielsweise gesättigte und ungesättigte Mono-, Di- und Tricarbonsäuren wie z.B. Ameisensäure, Essigsäure, Oxalsäure, Phthalsäure, Bernsteinsäure und Zitronensäure in Betracht.

Unter Halogen bei $R_2$ und $R_3$ sind Fluor, Chlor, Brom und Jod, insbesondere aber Chlor, zu verstehen.

Die für $R_2$ und $R_3$ in Frage kommenden Alkyl- oder Alkoxygruppen können geradkettig oder verzweigt sein. Beispiele solcher Gruppen sind u.a.: Methyl, Methoxy, Aethyl, Aethoxy, Propyl, Propoxy, Isopropyl, Isopropoxy, n-Butyl, n-Butoxy-, i-, sek.-, tert.-Butyl.

Wegen ihrer Wirkung bevorzugt sind Verbindungen der Formel I, worin $R_1$ Cyano und $R_2$ und $R_3$ je Wasserstoff oder Chlor bedeuten.

Die Verbindungen der Formel I werden nach an sich bekannten Methoden z.B. wie folgt hergestellt:

1)

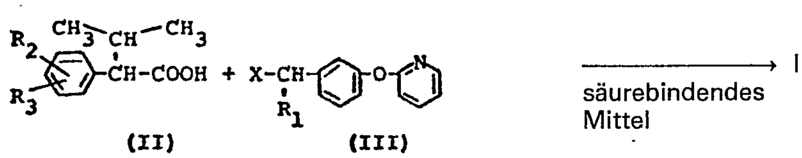


(II) (III)

2)

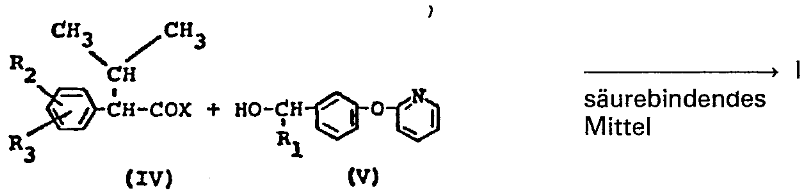


(IV) (V)

3)

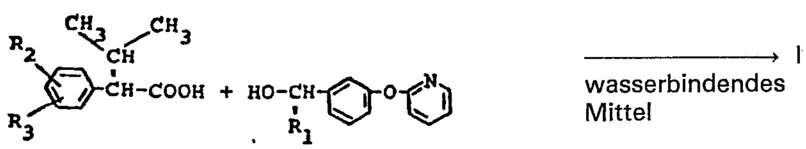


(II) (V)

4)

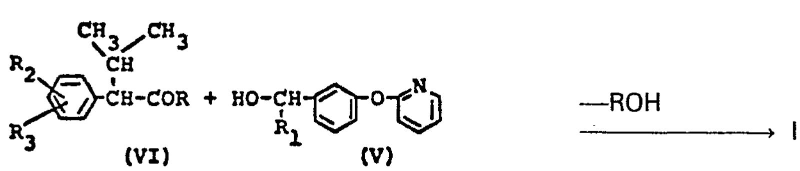


(VI) (V)

In den Formeln II bis VI haben $R_1$, $R_2$ und $R_3$ die für die Formel I angegebene Bedeutung.

In den Formeln III und IV steht X für ein Halogenatom, insbesondere Chlor oder Brom und in der Formel VI steht R für $C_1$—$C_4$-Alkyl, insbesondere für Methyl oder Aethyl. Als säurebindendes Mittel für die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen —10 und 120°C, meist zwischen 20 und 80°C bei normalen oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylol, Chloroform und Chlorbenzol; Nitrile wie Acetonitril; Dimethylsulfoxid und Ketone wie Aceton und Methyläthylketon.

Die Ausgangsstoffe der Formeln II, IV und VI sind bekannt wohingegen die Ausgangsstoffe der Formeln III und V neu sind. Alle diese Ausgangsstoffe können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonoptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpfalnzen, Insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine: Harnstoffe; andere pyrethrinartige Verbindungen sowie Karbamate und chlorierte Kohlenwasserstoff.

Mit besonderem Vorteil werden Verbindungen der Formel I auch mit Substanzen kombiniert, welche einen synergistischen oder verstärkenden Effekt ausüben.

Beispiel solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfonyl)-propyl)-benzol.

Verbindungen der Formel I können für sich allein oder zusammen mit geeigneten Träger-und/oder Zuschlagstoffen eingesetzt werden. Geeignete Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Bind- und/oder Düngemittel.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungs formen:

Stäubemittel, Streumittel, Granulate (Umhüllungsgranulate, Impragnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel 1 können beispielsweise wie folgt formuliert werden (Teile bedeuten Gewichtsteile):

*Stäubemittel:* Zur Herstellung eines a) 5%igen und b) 2%igen Stäubemittels werden die folgenden Stoffe verwendet:

a) 5 Teile Wirkstoff
95 Teile Talkum;
b) 2 Teile Wirkstoff
1 Teile hochdisperse Kieselsäure
97 Teile Talkum.

Der Wirkstoff wird mit den Trägerstoffen vermischt und vermahlen.

*Granulat:* Zur Herstellung eines 5%igen Granulates werden die folgenden Stoffe verwendet:

5 Teile Wirkstoff
0,25 Teile Epichlorhydrin
0,25 Teile Cetylpolyglykoläther
3,50 Teile Polyäthylenglykol
91 Teile Kaolin (Korngrösse 0,3—0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton in Vakuum verdampft.

*Spritzpulver:* Zur Herstellung eines a) 40%igen, b) und c) 25%igen, d) 10%igen Spritzpulver werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;
b) 25 Teile Wirkstoff
4,5 Teile Calcium-Ligninsulfonat
1,9 Teile Champagne-Kreide/Hydroxyäthylcellulose-Gemisch (1:1)
1,5 Teile Natrium-dibutyl-napthalinsulfonat
19,5 Teile Kieselsäure
19,5 Teile Champagne-Kreide
28,1 Teile Kaolin;
c) 25 Teile Wirkstoff
2,5 Teile Isooctylphenoxy-polyäthylen-äthanol
1,7 Teil Champagne-Kreide/Hydroxyäthylcellulose-Gemisch (1:1)
8,3 Teile Natriumaluminiumsilikat
16,5 Teile Kieselgur
46 Teile Kaolin;
d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen b) 25%igen und c) 50%igen emulgierbaren Konzentrates werden folgende Stoffe verwendet.

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;
b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol;
c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

*Sprühmittel:* Zur Herstellung eines a) 5%igen und b) 95%-igen Sprühmittels werden die folgenden Bestandteile verwendet:

a) 5 Teile Wirkstoff
1 Teil Epichlorhydrin
94 Teile Benzin (Siedegrenzen 160—190°C);

b) 95 Teile Wirkstoff
5 Teile Epichlorhydrin.

Beispiel 1.

*Herstellung von α,α-p-Chlorphenyl-iso-propyl-essigsäure-α'-cyano-3'-pyridyl-2'-oxybenzylester.*

7,5 g α,α-Chlorphenyl-isopropyl-essigsäurechlorid in 10 ml abs.Toluol werden bei 5°C zu einer Lösung von 7,5 g α-Cyano-3-(pyridyl-2'-oxi)-benzylalkohol und 2,7 g Pyridin in 100 ml als Toluol zugetropft. Das Reaktionsgemisch Wird 5 stunden bei Raumtemperatur gerührt. Dann wird das Gemisch in Eiswasser gegossen. Die organische Schicht wird mit 3%iger Salzsäure, Wasser, 3%iger Natriumbikarbonat-Lösung und wieder mit Wasser gewaschen. Nach dem Trocknen über Natriumsulfat und dem Abdestillieren des Toluols erhält man die Verbindung der Formel

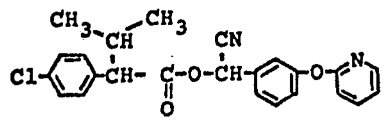

als viskose Flüssigkeit mit einer Refraktion von $n_D^{20°}=1,5656$.

Auf analoge Weise werden auch folgende Verbindungen hergestellt:

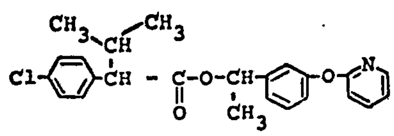

Smp.: 62—65°C

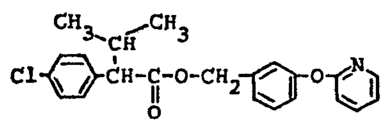

$n_D^{23°}=1,5712$

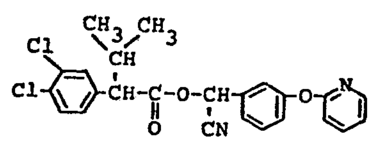

$n_D^{21°}=1,5719$

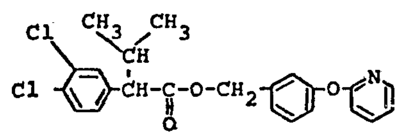

$n_D^{21°}=1,5734$

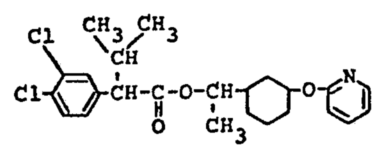

$n_D^{21°}=1,5672$

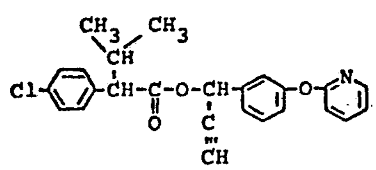

$n_D^{21°}=1,5700$

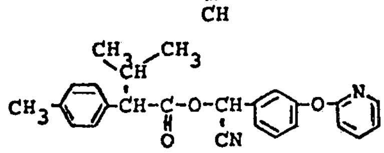

$n_D^{20°}=1,5605$

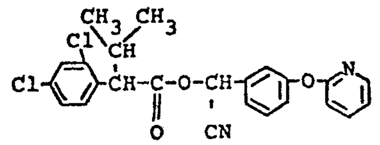

$n_D^{20°}=1,5688$

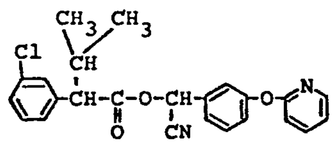

$n_D^{20°}=1,5670$

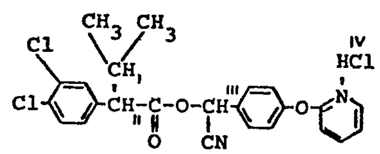

NMR—Daten
60 M Hz

2,3 ppm $H^{I}$ (m)
3,3 ppm $H^{II}$ (d)
6,4 ppm $H^{III}$ (d)
14,5 ppm $H^{IV}$ (s)

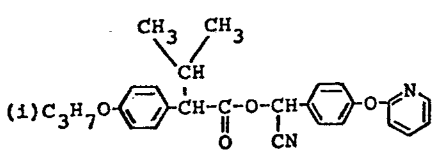

$n_D^{20°}=1,5516$

Beispiel 2.

A) *Insektizide Frassgift-Wirkung*

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

Beispiel 3.

*Wirkung gegen Chilo suppressalis*

Je 6 Reispflanzen der Sorte Caloro wurden in Plastiktöpfen, die einen oberen Durchmesser von 17 cm aufweisen, verpflanzt und zu einer Höhe von ca. 60 cm aufgezogen. Die Infestation mit Chilo suppressalis Larven ($L_1$: 3—4 mm lang) erfolgte 2 Tage nach der Wirkstoffzugabe in Granulatform (Aufwandmenge 8 kg Aktivsubstanz pro Hektare) in das Paddy-Wasser. Die Auswertung auf insektizide Wirkung erfolgte 10 Tage nach der Zugabe des Granulates.

Verbindungen gemäss Beispiel 1 wirkten im obigen Test gegen Chilo suppressalis.

Beispiel 4.

*Akarizide Wirkung*

Phaseolus vulgaris Pflanzen wurden 12 Stunden vor dem Test auf akarizide Wirkung mit einem infestierten Blattstück aus einer Massenzucht von Tetranychus urticae belegt. Die überglelaufenen beweglichen Stadien wurden aus einem Chromatographiezerstäuber mit den emulgierten Test-präparaten derart besprüht, dass kein Ablaufen der Spritzbrühe eintrat. Nach zwei bis 7 Tagen wurden Larven, Adulte und Eier unter dem Binokular auf lebende und tote Individuen ausgewertet und das Ergebnis in Prozenten ausgedrückt. Während der "Haltezeit" standen die behandelten Pflanzen in Gewächshauskabinen bei 25°C.

Verbindungen gemäss Beispiel 1 wirkten im obigen Test gegen Adulte, Larven und Eier von Tetranychus urticae.

Beispiel 5.

*Wirkung gegen Zecken*

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm

Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazinon). Verbindungen gemäss Beispiel 1 wirkten in diesen Tests gegen Adulte und Larven von Rhipicephalus bursa und sensible resp. OP-resistente Larven von Boophilus microplus.

**Patentansprüche**

1. Eine Verbindung der Formel

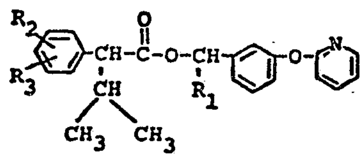

worin $R_1$ Wasserstoff, Cyano, Aethinyl oder Methyl und $R_2$ und $R_3$ je Wasserstoff, Halogen, $C_1$—$C_4$-Alkyl oder $C_1$—$C_4$-Alkoxy bedeuten.

2. Eine Verbindung gemäss Anspruch 1, worin $R_1$ Cyano und $R_2$ und $R_3$ je Wasserstoff oder Chlor bedeuten.

3. Die Verbindung gemäss Anspruch 2 der Formel

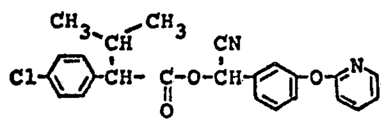

4. Die Verbindung gemäss Anspruch 2 der Formel

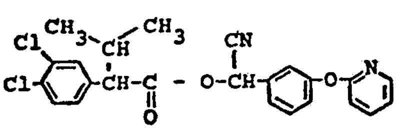

5. Die Verbindung gemäss Anspruch 2 der Formel

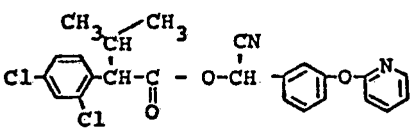

6. Ein Verfahren zur Herstellung einer Verbindung gemäss Anspruch 1, dadurch gekennzeichnet, dass man eine Verbindung der Formel

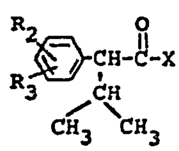

in Gegenwart eines säurebindenden Mittels mit einer Verbindung der Formel

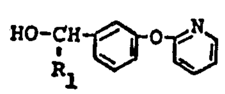

umsetzt, worin $R_1$, $R_2$ und $R_3$ die im Anspruch 1 angegebene Bedeutung haben und X für ein Halogenatom steht.

7. Ein Schädlingsbekämpfungsmittel, welches als aktive Komponente eine Verbindung gemäss Anspruch 1 und geeignete Träger und/oder andere Zuschlagstoffe enthält.

8. Verwendung einer Verbindung gemäss Anspruch 1 zur Bekämpfung von verschiedenartigen tierischen und pflanzlichen Schädlingen.

9. Verwendung einer Verbindung gemäss Anspruch 1 zur Bekämpfung von Insekten und Vertretern der Ordnung Akarina.

**Revendications**

1. Un composé de formule

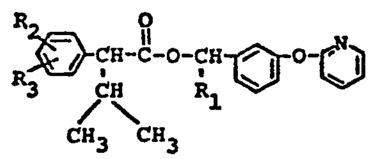

dans laquelle $R_1$ représente l'hydrogène, un groupe cyano, éthynyle ou méthyle et $R_2$ et $R_3$ représentent chacun l'hydrogène, un halogène, un groupe alkyle en $C_1$—$C_4$ ou alcoxy en $C_1$—$C_4$.

2. Un composé selon la revendication 1, dans lequel $R_1$ représente le groupe cyano et $R_2$ et $R_3$ représentent chacun l'hydrogène ou le chlore.

3. Le composé selon la revendication de formule

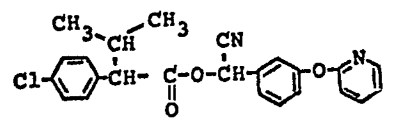

4. Le composé selon la revendication 2, de formule

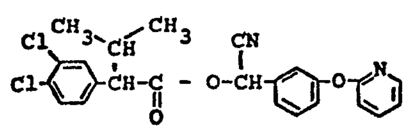

5. Le composé selon la revendication 2, de formule

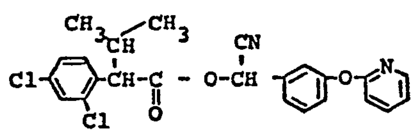

6. Un procédé de préparation d'un composé selon la revendication 1, caractérisé en ce que l'on fait réagir un composé de formule

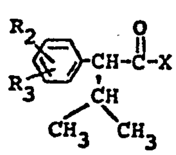

en présence d'un agent fixant les acides, avec un composé de formule

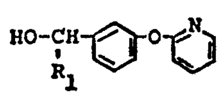

les symboles $R_1$, $R_2$ et $R_3$ ayant les significations indiquées dans la revendication 1 et X représentant un atome d'halogène.

7. Un produit pesticide contenant en tant que composant actif un composé selon la revendication 1 avec des véhicules et/ou d'autres additifs appropriés.

8. Utilisation d'un composé selon la revendication 1 dans la lutte contre les parasites animaux et végétaux de types variés.

9. Utilisation d'un composé selon la revendication 8 dans la lutte contre les insectes et les représentants de l'ordre des acariens.

**Claims**

1. A compound of the formula

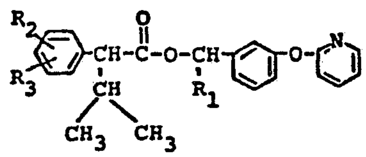

wherein $R_1$ represents hydrogen, cyano, ethynyl or methyl, and $R_2$ and $R_3$ each represent hydrogen, halogen, $C_1$—$C_4$-alkyl or $C_1$—$C_4$-alkoxy.

2. A compound according to Claim 1, wherein $R_1$ represents cyano, and $R_2$ and $R_3$ each represent hydrogen or chlorine.

3. The compound according to Claim 2 of the formula

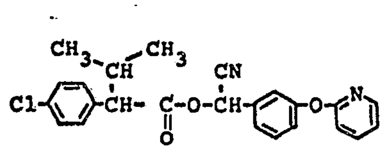

4. The compound according to Claim 2 of the formula

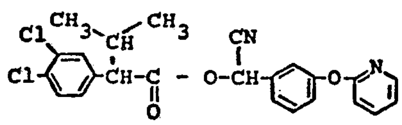

5. The compound according to Claim 2 of the formula

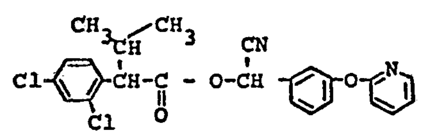

6. A process for producing a compound according to Claim 1, which process comprises reacting a compound of the formula

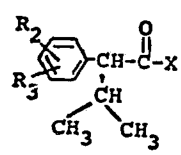

in the presence of an acid-binding agent, with a compound of the formula

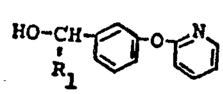

wherein $R_1$, $R_2$ and $R_3$ have the meanings given in Claim 1, and X represents a halogen atom.

7. A pesticidal composition which comprises a compound according to Claim 1 as active ingredient, and suitable carriers and/or other additives.

8. A method of combating various animal and plant pests at a locus, which method comprises applying to the locus a compound as claimed in claim 1.

9. A method according to claim 8 for combating insects, and members of the order *Acarina.*